# FASCICULE DE BREVET EUROPEEN

(11) **EP 1 434 565 B1**
(45) Date de publication et mention de la délivrance du brevet: **07.12.2016**
(21) Numéro de dépôt: 02795309.0
(22) Date de dépôt: 11.10.2002
(51) Int. Cl.: A61K 8/63, A61Q 19/00, A61Q 19/06, A61Q 19/08, A61K 8/97, A61K 31/56, A61K 36/074

(54) **UTILISATION D'ACIDES GANODERIQUES EN TANT QU'AGENTS COSMETIQUES**
VERWENDUNG VON GANODERICSAÜREN ALS KOSMETISCHE AGENTIEN
USE OF GANODERIC ACIDS AS COSMETIC AGENTS

(30) Priorité: 11.10.2001 FR 0113305
(43) Date de publication de la demande: 07.07.2004
(73) Titulaire: YVES SAINT LAURENT PARFUMS, (Société Anonyme), 92200 Neuilly sur Seine (FR)
(72) Inventeur: GUESNET, Joëlle, F-91440 Bures sur Yvette (FR); GUEZENNEC (née LOUCEL), Anne, Bruna, Suzanne, F-78290 Croissy (FR)
(74) Mandataire: Martin, Didier Roland Valéry
(86) Numéro de dépôt international: PCT/FR2002/003494
(87) Numéro de publication internationale: WO 2003/030811

(56) Documents cités:
- EP-A- 0 591 603
- EP-A- 1 092 765
- WO-A-98/13512
- DE-A- 19 911 679
- DATABASE CAPLUS [en ligne] CHEMICAL ABSTRACTS SERVICE, COLUMBUS, OHIO, US; retrieved from STN Database accession no. 1997:469964 XP002203149 & JP 09 143025 A (ARSOA OSHO KK) 3 juin 1997 (1997-06-03)
- DATABASE WPI Week 200009 Derwent Publications Ltd., London, GB; AN 2000-098262 XP002203150 & CN 1 230 369 A (LIZZ-I)
- ANONYME: ARTICLE INTERNET, [en ligne] XP002235871 Extrait de l'Internet: <URL:http://www.thaifloriade.thaigov.net/h ort_cd/herb/html/16_Rcishi%20musroom.htm> [extrait le 2003-03-25]
- ANONYME: ARTICLE INTERNET, [en ligne] pages 1-17, XP002235872 Extrait de l'Internet: <URL:http://www.fruiting-bodies.co.uk/canc er_research/cr_chapter3.htm> [extrait le 2003-03-25]
- MIN B.-S. ET AL: "Triterpenes from the Spores of Ganoderma lucidum and Their Inhibitory Activity against HIV-1 Protease", CHEM. PHARM.BULL., vol. 46, no. 10, 1998, pages 1607-1612, XP001098588,
- KOMODA Y. ET AL: "Ganoderic acid and its derivatives as cholesterol synthesis inhibitors", CCHEM. PHARM. BULL., vol. 37, no. 2, 1989, pages 531-533, XP001538276,
- MIN ZHU ET AL: "Triterpene Antioxidants from Gandoderma lucidum", PHYTOTHERAPY RESEARCH, vol. 13, 1999, pages 529-531,

## Description

### DOMAINE TECHNIQUE

La présente invention se rapporte aux domaines cosmétique et médical en relation avec les processus et pathologies associés au vieillissement des mammifères, et en particulier de l'être humain, et à des produits ou nouvelles utilisations susceptibles de traiter ou prévenir l'apparition des phénomènes naturels du vieillissement, et en particulier du vieillissement cutané.

La présente invention concerne l'utilisation de substances en tant qu'agents cosmétiques, ainsi que l'utilisation de substances pour la préparation d'un médicament en vue du traitement ou de la prévention d'un désordre cutané.

L'invention concerne également une composition comprenant un mélange de plusieurs espèces d'acides ganodériques, ainsi que plus généralement une nouvelle utilisation d'acides ganodériques.

### TECHNIQUE ANTERIEURE

L'effet anti-vieillissement de la DHEA (DéHydroEpiAndrostérone) et de son dérivé sulfate est déjà largement connu chez l'être humain. On sait en particulier que la DHEA et son dérivé sulfate circulent en quantité importante chez l'adulte et que, parmi les effets décrits concernant cette substance, on connaît ceux s'opposant aux processus et pathologies associés au vieillissement.

On sait également que la production de DHEA voit son taux diminuer chez l'être humain en fonction de l'âge, cette diminution pouvant permettre d'expliquer, au moins en partie, les pathologies associés au vieillissement. Ainsi, on peut tenter d'expliquer les effets anti-vieillissement observés chez l'individu adulte à la suite d'une supplémentation médicamenteuse à base de DHEA, par le rétablissement au niveau de l'individu de l'équilibre chimique entre le taux de DHEA et le taux d'hormones corticoïdes (cortisol, cortisone). En effet, on sait que la DHEA et le cortisol sont produits à partir d'un même précurseur, la prégnénolone, et que la diminution de la production de la DHEA et de son dérivé sulfate est directement corrélée au vieillissement, puisque la diminution du taux de DHEA observée chez les personnes âgées déséquilibre le rapport taux de DHEA / taux d'hormones corticoïdes, au profit de ces dernières dont la production reste constante quel que soit l'âge. On aboutit ainsi à une expression dominante des effets des hormones corticoïdes, ce qui se traduit par des observations et phénomènes connus tel que : l'atrophie cutanée, la fonte musculaire, la redistribution des graisses, l'immuno-suppression, l'ostéoporose etc., toutes ces manifestations étant classiquement associées au vieillissement humain. En définitive, on pense que les effets anti-vieillissement de la DHEA seraient donc attribués à une activité anti-glucocorticoïde.

Il est néanmoins connu que la DHEA se situe au carrefour d'une voie métabolique pouvant conduire à la formation d'hormones stéroïdiennes, androgènes ou oestrogènes. L'utilisation, à titre de supplémentation médicamenteuse, de la DHEA ou de son dérivé sulfate, peut donc être à l'origine de l'apparition d'effets secondaires indésirables chez certains individus, et notamment chez la femme. Ceci constitue une limite à l'utilisation générale de la DHEA ou de son dérivé sulfate, utilisation qui est de ce fait réglementée et limitée au domaine médical, ce qui exclut notamment son utilisation dans le domaine de la cosmétique.

Les processus et pathologies associés au vieillissement humain sont par ailleurs connus, mais seulement en partie élucidés. Cet aspect prend désormais une grande importance et on recherche constamment de nouvelles substances ou de nouvelles utilisations susceptibles de traiter ou de prévenir ce phénomène naturel chez l'homme ou la femme, tout en ne créant pas d'effets secondaires indésirables.

On connaît déjà par ailleurs le champignon *Ganoderma lucidum* de la famille des *ganodermataceaes* (polyporacés), dont les propriétés bénéfiques pour la santé ont été largement rapportées dans la littérature. Cette espèce de champignon, bien connue en Chine depuis 2 000 ans environ pour ses propriétés curatives de toutes sortes, peut être aujourd'hui obtenue par culture sur de la sciure de bois humide, après avoir été longtemps difficile à trouver à l'état naturel.

Les propriétés médicales rapportées sont extrêmement variées, et ont été rapportées dans de nombreuses publications, et notamment par exemple dans *Medicinal Benefits of the Mushroom Ganoderma (Advances in microbiology, 1992)*. A titre d'exemples non limitatifs, on peut citer des propriétés rapportées telles que : une action anti-tumorale, une action d'amélioration de la synthèse protéique, de la synthèse d'acides nucléiques, des actions de protection du foie et des actions détoxiquantes, une action de régulation du système cardio-vasculaire, une action sur le système respiratoire et une action amélioratrice sur le système immunitaire. D'autres propriétés sont également rapportées telles que : diurétique, régulateur du transit intestinal, antitussif, anti-hypertenseur, etc.

On sait également que le champignon *ganoderma* est une source particulièrement intéressante de composés triterpènes.

La plupart des études sur la composition chimique des champignons *ganoderma* ont été entreprises sur l'espèce *ganoderma lucidum,* et ces études font état d'un très grand nombre de constituants chimiques (*Encyclopedia of Common Natural Ingredients, Second Edition, Albert Y. LEUNG, Steven FOSTER)* tels : stérol, enzymes diverses (*lactase, cellulase, amylase*), protéines solubles dans l'eau, polypeptides, acides aminés, sucres divers, alcane, acides gras, polysaccharides et éléments inorganiques divers, composés triterpènes incluant toutes les espèces d'acides ganodériques connues, ainsi que des acides lucidéniques notamment, mais aussi des acides ganodermiques, des lucidones, des acides ganodéréniques, des ganodérals et ganodérols.

La plupart des études pharmacologiques ont été effectuées sur des extraits divers de *ganoderma lucidum,* et on rapporte que les propriétés cardio-vasculaires hypotensives sont à mettre en relation avec les composés triterpènes du champignon *ganoderma lucidum,* incluant les acides ganodériques B, D, F, H, K, S et Y, l'acide ganodérique F étant le plus actif. On a également observé que les effets anti-histaminiques du champignon pouvaient être dus aux acides ganodériques C et D.

De la même façon, la littérature rapporte que les effets anti-tumoraux peuvent être reliés à différents composants du champignon, et notamment à des acides ganodériques T et Z.

On rapporte également que les acides ganodériques extraits du champignon *ganoderma* ont été utilisés en tant que suppléments alimentaires ou énergétiques.

Les utilisations d'extraits de champignon *ganoderma* sont diverses et telles que la médecine traditionnelle, l'alimentation spécialisée à caractère biologique ou énergétique. On rapporte également l'utilisation d'extraits de champignon *ganoderma* dans le domaine de la cosmétique, ces extraits étant utilisés dans des produits pour soins de la peau (crèmes ou lotions) pour leurs propriétés d'hydratation, de nutrition et de blanchiment notamment.

### EXPOSE DE L'INVENTION

L'objet assigné à l'invention vise précisément à remédier aux différents problèmes évoqués précédemment, en relation avec les limites à l'utilisation de la DHEA pour lutter contre le vieillissement, et à trouver une voie nouvelle pour traiter ou prévenir le vieillissement.

Un autre objet de l'invention vise à trouver une nouvelle voie pour traiter ou prévenir l'apparition du vieillissement cutané.

Un autre objet de l'invention vise à proposer une nouvelle utilisation d'une composition à base d'acides ganodériques.

Un autre objet de l'invention vise à proposer une nouvelle utilisation médicale.

Un autre objet de l'invention vise à proposer une nouvelle composition à base d'acides ganodériques particulièrement efficaces en tant qu'agents contre le vieillissement.

Les objets assignés à l'invention sont atteints à l'aide de l'utilisation de composés ayant une structure triterpénoïde conforme à l'objet de la revendication 1.

Les objets assignés à l'invention sont atteints à l'aide de l'utilisation d'une composition d'acides ganodériques pour la préparation d'un médicament, en vue du traitement ou de la prévention d'un désordre cutané, conforme à l'objet de la revendication 7.

Les objets assignés à l'invention sont atteints à l'aide d'une composition comprenant des acides ganodériques conforme à l'objet de la revendication 10.

### MEILLEURE MANIERE DE REALISER L'INVENTION

D'une manière surprenante, il a ainsi pu être mis en évidence la nouvelle utilisation de composés ayant une structure triterpénoïde avec deux fonctions méthyles en position 4 de formule : 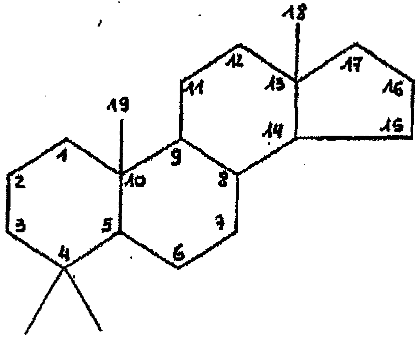 et comportant au moins une fonction hydroxyle en positions 3 et 7 en tant qu'agent cosmétique.

Ces composés se sont en effet révélés avoir des effets anti-vieillissement généraux sur l'être humain, lorsqu'ils sont utilisés en tant qu'agents cosmétiques sous des formes diverses.

A titre d'exemple, les effets anti-vieillissement dans le domaine cosmétique peuvent s'exercer sur le vieillissement cutané en le retardant ou en le prévenant.

De manière générale, les effets anti-vieillissement constatés sont des effets protecteurs ou retardateurs sur l'apoptose cortico-induite, qui constitue un modèle d'étude représentatif des effets liés à la présence des glucocorticoïdes.

A côté des effets anti-vieillissement, il s'est avéré que les composés ayant au moins une fonction hydroxyle en positions 3 et 7 et utilisés en tant qu'agents cosmétiques, avaient également des effets amincissants localisés résultant d'une prise en charge des surcharges graisseuses localisées.

Parmi les composés connus ayant une structure triterpénoïde ou encore un squelette triterpène, tels que ceux visés précédemment dans la présente demande, les acides ganodériques se sont révélés particulièrement intéressants.

Il s'est avéré que les acides ganodériques ont des effets anti-vieillissement en général sur l'être humain, et que ces propriétés générales anti-vieillissement pouvaient être reliées à des effets anti-glucocorticoïdes en général.

**TABLEAU 1 :**

| Relation Structure - Activité anti-glucocorticoïde contre modèle de protection sur l'apoptose cellulaire cortico-induite | | | | | | | | | |
|---|---|---|---|---|---|---|---|---|---|
| | | | | | | | | | |
| | | | | | | | | | |
| **Echantillon** | **Acides ganodériques** | **Acides ganodériques (%)** | | | | | | **B+C2+G totaux** | **% de cellules en apoptose cortico-induite** |
| | **totaux (%)** | A | B | C₁ | C₂ | F | G | | |
| 1 | 10,26 | 3 | 1,83 | 2,35 | 1,03 | 1,07 | 0,98 | 37,40% | 37 |
| 2 | 8,4 | 1,81 | 1,86 | 1,75 | 0,68 | 0,97 | 1,33 | 46,00% | 38 |
| 3 | 26,72 | 8,02 | 4,09 | 9,6 | - | 5,01 | - | 15,30% | 90 |

Les essais menés, dont les résultats figurent au tableau 1, ont permis de mettre en évidence que les effets anti-vieillissement observés sont des effets protecteurs sur l'apoptose cortico-induite.

Les acides ganodériques utilisés peuvent provenir de toute source envisageable, qu'il s'agisse d'acides ganodériques obtenus par synthèse ou par extraction à partir d'une substance ou d'un élément naturel.

De manière avantageuse, les acides ganodériques proviennent d'un extrait de champignon de la famille des *ganodermataceaes* (polyporacés).

Dans les essais menés (tableau 1), l'extrait de champignon testé provient de l'espèce *ganoderma lucidum,* étant entendu qu'au sens de l'invention, tout extrait d'acide ganodérique utilisable peut provenir des autres espèces connues de *ganoderma,* et par exemple de *ganoderma japonicum,* de *ganoderma capense* et de *ganoderma applanatum* notamment.

Les essais ont été réalisés avec des échantillons d'acides ganodériques se présentant sous une forme d'extrait poudreux, les essais ayant pour but d'évaluer les effets d'actifs cosmétiques sur l'apoptose induite par des glucocorticoïdes, sur des thymocytes de souris.

L'apoptose est induite par la déxaméthasone et analysée par mesure de la quantité de caspase 3 intracellulaire, marqueur spécifique de l'apoptose par cytométrie de flux.

Les thymocytes sont prétraitées ou non par les produits, pendant une heure, avant l'induction de l'apoptose. Les cellules sont ensuite cultivées pendant dix-huit heures, des contrôles sans déxaméthasone permettant d'évaluer un éventuel effet apoptotique direct des produits aux plus fortes concentrations.

Les thymocytes sont ensuite centrifugées et incubées en présence d'anti-caspase 3-phycoerythrine et fixées en PBS/paraformaldéhyde puis analysées par cytométrie. Les résultats sont exprimés en pourcentage de cellules marquées (caspase +) en triplicate, sur trois échantillons différents.

Les essais réalisés ont donc permis de montrer que les échantillons contenant des acides ganodériques présentaient un effet protecteur marqué, notamment à un niveau de concentration de l'ordre de 3-10⁻⁶%.

Il s'est avéré que parmi toutes les espèces d'acides ganodériques présentes dans les *ganoderma* et dont la formule générale est la suivante : 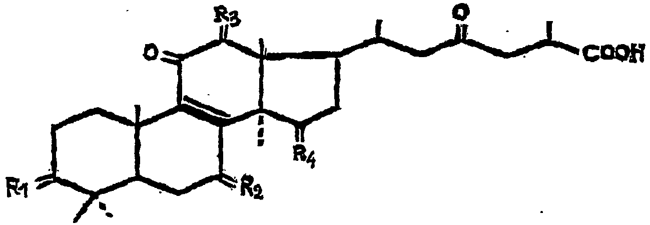 les dérivés B, C₂ et G dont la formule est la suivante : 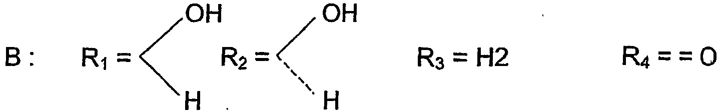 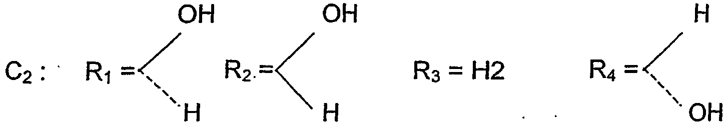 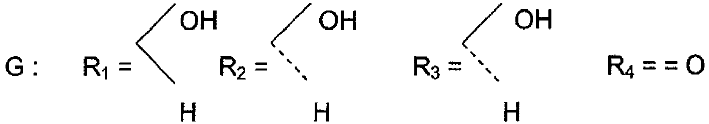 présentaient une activité particulièrement intéressante en tant qu'agents cosmétiques, en vue d'obtenir des effets anti-vieillissement, notamment sur l'apoptose cortico-induite, ainsi que des effets amincissants localisés.

Ces activités surprenantes sont à mettre en relation avec l'existence de deux fonctions hydroxyles en position 3 et 7 présentes dans chacune des fractions d'acides ganodériques B, C₂ et G. On pense en effet que le radical 3-hydroxylé est directement associé à une action anti-vieillissement, alors que le radical 7-hydroxylé aura une action de verrouillage de la production de précurseurs hormonaux, en verrouillant notamment la transformation de la DHEA.

L'invention concerne donc également la nouvelle utilisation d'une composition d'acides ganodériques pour la préparation d'un médicament, en vue du traitement ou de la prévention d'un désordre cutané. Avantageusement, cette nouvelle utilisation consiste à traiter ou prévenir l'apoptose cortico-induite.

Les effets anti-glucocorticoïdes mis en évidence *in vitro* peuvent s'exercer sur d'autres cibles telles que l'inflammation ou le métabolisme adipocytaire, de telle sorte que l'invention porte également sur une nouvelle utilisation des acides ganodériques, qui consiste à traiter ou prévenir des désordres résultant d'inflammations cutanées ou du métabolisme adipocytaire.

Le tableau 1 montre que parmi les différents extraits d'acides ganodériques utilisés, il apparaît que seuls les extraits de champignon (ganoderma lucidum dans les exemples) comprenant au moins une fraction d'acide ganodérique B et/ou au moins une fraction d'acide ganodérique C₂ et/ou au moins une fraction d'acide ganodérique G, sont susceptibles d'avoir un effet protecteur sur l'apoptose cortico-induite.

les extraits d'acides ganodériques utilisés comprennent un mélange d'acides ganodériques choisis parmi les espèces B, C₂, G, seuls ou en combinaison.

En effet, il s'est avéré que les extraits d'acides ganodériques utilisés et dépourvus du mélange des acides ganodériques B, C₂ et G ou de l'un d'eux au moins, par exemple au profit des acides ganodériques A, C₁ et F, ne permettaient pas de mettre en évidence une activité anti-glucocorticoïde réellement significative.

On pense donc bien que l'effet anti-vieillissement mis en évidence est lié à la présence d'un autre radical 3-hydroxylé, qui lui est absent des espèces d'acides ganodériques A, C₁ et F.

Il a également pu être mis en évidence que l'activité anti-vieillissement ou de traitement ou de prévention d'un désordre cutané pouvait être d'autant plus efficace que la proportion d'acides ganodériques B, C₂ et G était importante au sein de la fraction totale des acides ganodériques apportés.

Le tableau 1 permet également de constater que l'activité anti-glucocorticoïdes des fractions ou extraits d'acides ganodériques semble directement liée à la proportion d'acides ganodériques B, C₂ et G au sein de la fraction totale des acides ganodériques. L'échantillon 3 montre une activité anti-glucocorticoïdes réduite, comparativement aux échantillons 1 et 2 dont la proportion d'acides ganodériques B, et/ou C₂, et/ou G est supérieure au sein de la fraction totale des acides ganodériques.

Il semble donc que la proportion d'acides ganodériques B et/ou C₂ et/ou G est d'autant plus efficace que cette proportion est supérieure à 15% au sein de la fraction totale des acides ganodériques, et de préférence par exemple au moins égale à 20 ou 30%.

L'invention vise donc à protéger une composition selon la revendication 11 comprenant des acides ganodériques dans laquelle ladite composition comprend au moins une fraction d'acides ganodériques B et/ou d'acides ganodériques C₂ et/ou d'acides ganodériques G, ladite ou lesdites fractions étant supérieures à 15% au sein de la fraction totale des acides ganodériques.

La composition selon l'invention pourra donc comprendre un mélange d'au moins deux fractions différentes d'acides ganodériques B et/ou C₂ et/ou G.

Avantageusement, la composition selon l'invention comprend un mélange d'acides ganodériques B, C₂ et G, et dont la proportion est supérieure à 15% au sein de la fraction totale d'acides ganodériques.

Au sens de l'invention, les extraits de champignon, notamment ceux provenant du *ganoderma lucidum,* pourront être utilisés sous forme purifiée à 100% ou sous fraction enrichie par extraction ou solvants divers.

Dans le cas d'utilisation d'acides ganodériques obtenus par synthèse, les extraits pourront être utilisés sous forme pure ou diluée.

Les applications envisagées concernent bien évidemment l'utilisation dans le domaine de la cosmétique, et également le domaine de la dermatologie, les applications envisagées pouvant être intégrées directement lors de la formulation de crèmes, de sérum, d'émulsions, de gemmes, etc.

L'invention vise donc également à protéger une composition selon l'invention qui est formée par une composition cosmétique comprenant les différents excipients appropriés pour une utilisation cosmétique.

Les différents excipients appropriés pour une telle utilisation sont bien connus de l'homme du métier et figurent dans les différentes formulations fournies ci-après, et qui concernent une crème anti-rides, un sérum anti-tâches, un fond de teint, une émulsion blanchissante protectrice, un gel amincissant et une crème hydratante, sans que cette liste puisse être pour autant considérée comme étant limitative.

L'invention vise également à protéger une composition selon l'invention qui est formée par une composition alimentaire qui comprend les différents excipients appropriés pour une utilisation alimentaire, de tels excipients étant bien connus de l'homme du métier.

De manière générale, les excipients pour utilisation alimentaire pourront être tirés des exemples suivants :
Composition de gélules / capsules pour complément alimentaire :
   Enveloppe :
      - Gélatine,
      - Glycérol,
      - Eau purifiée,
      - Colorant autorisé alimentaire (oxyde de fer...).
   Poudre :
      - Extrait de *Ganoderma,*
      - Lactose (ou dextrine / maltose) qs.
   Autre composition alimentaire
      - Eau,
      - Huile végétale (maïs, soja),
      - Emulsifiant (mono-diglycérides d'acides gras),
      - Epaississant (alginate de sodium, gomme guar ou xanthane),
      - Extrait de *Ganoderma,*
      - Stabilisant (phosphate disodique).

Enfin, l'invention vise également à protéger une composition qui est formée par une composition pharmaceutique comprenant les excipients appropriés pour une utilisation pharmaceutique, les différents excipients étant eux aussi bien connus de l'homme du métier.

De manière générale, les excipients pour utilisation pharmaceutique pourront être tirés des exemples suivants :
Compositions pharmaceutiques pour application topique :
   Emulsion E/H n°1 :
      - Eau,
      - Glycérine codex,
      - Huile paraffine codex,
      - Stéarate de glycérol,
      - Polymère siliconé,
      - Polymère carboxylique,
      - Triéthanolamine.
   Emulsion E/H n°2 :
      - Eau,
      - Dioctylcyclohexane,
      - Isopropyl myristate,
      - Glycéryl stéarate,
      - PEG7 hydrogenated castor oil,
      - Glyceryl sorbitan stearate,
      - Cyclomethicone,
      - Phenoxyethanol,

A titre d'exemple non limitatif, des fractions d'acides ganodériques B, C₂ et G, seules ou en mélange, peuvent être utilisées dans les formulations suivantes de : crème anti-rides, sérum anti-taches, fond de teint, émulsion blanchissante protectrice contre les UVA et contre les UVB, gel amincissant et crème hydratante.

La composition comprenant un mélange d'un ou de plusieurs acides ganodériques B, C₂ et G présents à raison d'au moins 15% au sein de la fonction totale des acides ganodériques peut être formée par une composition alimentaire, et par exemple du genre composition énergétique se présentant sous toute forme et conditionnement approprié, et par exemple sous une forme solide ou liquide ou pâteuse ou semi-pâteuse.

Des essais complémentaires, tels que ceux figurant ci-après, ont été entrepris pour démontrer le parallélisme d'action existant entre la DHEA et l'action des acides ganodériques B, C₂ et G, seuls ou en combinaison, pour lutter contre le vieillissement cutané, ces résultats démontrant donc que les acides ganodériques B, C₂ et G, du fait de leur proximité de structure chimique avec la DHEA, liée notamment à la présence d'un radical 3-hydroxylé, peuvent être considérés comme des substituts à la DHEA pour lutter contre le vieillissement cutané.

Les essais figurant au tableau 2 ci-après concernent la régulation d'une enzyme, la MMP1 (collagénase interstitielle humaine) appartenant aux métalloprotéinases, de la famille des protéases, enzymes dégradant la matrice extra-cellulaire, la MMP1 dégradant particulièrement le collagène natif.

Il est déjà connu que la stimulation des métalloprotéinases entraîne la dégradation de la matrice extra-cellulaire, le processus de dégradation pouvant être accéléré par divers stimuli, et notamment lors de l'exposition à des rayons ultraviolets, ou lors de phénomènes inflammatoires. Ces effets sont largement décrits dans la bibliographie, et sont connus comme participant au processus général de vieillissement, et notamment cutané.

Les essais, dont les résultats figurent au tableau 2, ont donc consisté à comparer l'activité de la DHEA et des acides ganodériques B, C₂ et G sur la régulation de l'activité de la MMP1 après stimulation des fibroblastes par PMA.

Les résultats montrent le parallélisme existant dans l'activité de prévention du désordre cutané se manifestant par une prévention relativement à l'infection, ou donc au vieillissement cutané.

Les effets anti-vieillissement se manifestent pour chacun des acides ganodériques B, C₂ et G, ce dernier semblant présenter une activité supérieure aux deux autres acides ganodériques.

Plus précisément, le tableau 2 de résultats montre que la DHEA comme les acides ganodériques permettent de réguler la quantité de MMP1 après une stimulation des cellules. En effet, sous stimulation inflammatoire (traitement au PMA), la quantité de MMP1 présente dans les cellules est augmentée de sa valeur basale (contrôle - PMA) à une valeur arbitrairement fixée comme référentiel témoin à 100% (témoin + PMA). Lors du traitement des cellules par la DHEA (DHEA 0,001%) cette valeur est ramenée à 60% de la valeur du témoin (témoin + PMA) de façon statistiquement significative (p<0,01), ce qui représente une diminution de la quantité de MMP1 d'environ 40%. Cet effet inhibiteur est également retrouvé de façon significative avec les acides ganodériques pour lesquels la diminution de la quantité MMP1 est d'environ 30% pour les acides ganodériques B et C₂, et atteint plus de 40% pour l'acide ganodérique G.

### Essais complémentaires :

### MATERIELS ET METHODES UTILISES EXPERIENCES MMP-1/FIBROBLASTES

### a) Cellules

- Fibroblastes humains normaux profonds (type R₂Ad₂), utilisé au 2e passage.
- Culture: 37° C, 5 % CO₂
- Milieu de croissance : milieu Tebu PM-1
- Milieu d'essai : DMEM/HAMF10, vol/vol (Invitrogen 21969035 / Invitrogen 31550-023)
   Hepes 15 mM (Invitrogen 1563-056)
   L-glutamine 2 mM (Invitrogen 25030024)
   pénicilline 50 UI/ml streptomycine 50 µg/ml (Invitrogen 15070063)
   **sans sérum de veau foetal**

### b) Produits à l'essai

### DHEA

- solution stock : 10 % dans l'éthanol puis dilution à 0.1 % dans une solution de BSA délipidée à 5 mg/ml final
- dilutions : en milieu de culture stérile
- **concentration finale testée: 0.001** % (50 µg/ml BSA 1^{e} conc, final) Acide Ganodérique B
- solution stock : 10 % en DMSO
- dilutions : en milieu de culture stérile
- **concentration finale testée: 0.04** % (50 µg/ml BSA final) Acide Ganodérique C2
- solution stock : 10 % en DMSO
- dilutions : en milieu de culture stérile
- **concentration finale testée: 0.04 %** (50 µg/ml BSA final) Acide Ganodérique G
- solution stock : 10 % en DMSO
- dilutions : en milieu de culture stérile
- **concentration finale testée: 0.04 %** (50 µg/ml BSA final)

### c) Essai ; mise au point des conditions

Les cellules ont été ensemensées à 17000 cellules par puits, en plaques 96 puits, une plaque pour chaque temps de culture. Toutes les conditions en triplicata (3 puits de culture). Les cellules ont été pré-cultivées pendant 24 h en milieu sans SVF. Après 24 h de pré-culture, les milieux ont été remplacés par un milieu équivalent contenant) les produits à l'essai ou non (contrôles) ou la DHEA à 0.001 %. Après 4 h d'incubation, une solution 10 fois concentrée de « phorbol myristate acétate » (PMA, Sigma, 0.1 µg/ml final) a été ajoutée ou non (contrôles) et les cellules ont été cultivées pendant 6 h supplémentaires.

A la fin de chaque temps de culture, les milieux de culture ont été récupérés et congelés à -80 °C pour dosage de la quantité de MMP-1 et la viabilité des tapis cellulaires a été contrôlée en utilisant un test standard de réduction du MTT.

Le dosage de MMP-1 (collagénase interstitielle humaine) a été réalisé par ELISA, à l'aide d'un kit « BIOTRACK human MMP-1, ELISA system, Amersham RPN2610 », selon les directives du fournisseur.

Résumé des conditions testées :
Modèle : fibroblastes humains
Test : dosage quantité de MMP-1 produite (protéine, ELISA)
Milieux : sans sérum
Tests : produits à l'essai, DHEA 0.001 % ou contrôles non traités, pendant 4 h
Stimulation : ajout de PMA (ou non, contrôles) et culture pendant 6 h. Produits (selon viabilités sur fibroblastes):
   - A.G. B 0:04 % ;
   - A.G. C2 0.04 % ;
   - A.G. G 0.04 % ;
   - DHEA 10 µg/ml

### Traitement des données

Les données brutes de comptage ont été transférées et traitées sous le logiciel PRISM® (Graph Pad Software).

Les comparaisons inter-groupes ont été réalisées par analyse de variance (ANOVA) à l'aide du test de comparaison multiple de Dunnett.

**TABLEAU 2 :**

| **DHEA : Dosage activité MMP1 (+PMA) à 6h** | | | | | |
|---|---|---|---|---|---|
| **Traitement** | **MMP1** (**ng**/**ml**) | **Ecart**-**type** | **n** | **% témoin +PMA** | **p** |
| **Témoin + PMA** | 10,54 | 1,56 | 3 | 100 | - |
| **Contrôle - PMA** | 3,61 | 0,76 | 3 | 34 | p<0,01 |
| **DHEA (0,001%)** | 6,38 | 0,73 | 3 | 61 | p<0,01 |

| **Acides Ganodériques : Dosage activité MMP1 (+ PMA) à 6h** | | | | | |
|---|---|---|---|---|---|
| **Traitement** | **MMP1** (**ng**/**ml**) | **Ecart**-**type** | **n** | **% témoin +PMA** | **p** |
| **Témoin + PMA** | 17,89 | 2,48 | 3 | 100 | - |
| **Contrôle - PMA** | 7,67 | 1,23 | 3 | 43 | p<0,01 |
| **A. ganodérique B (0,04%) A. ganodérique C2 (0,04%)** | 13,52 | 2,21 | 3 | 76 | p<0,05 |
| | 12,95 | 1,74 | 3 | 72 | p<0,01 |
| **A. ganodérique G (0,04%)** | 10,15 | 0,82 | 3 | 57 | p<0,01 |

**CREME ANTI-RIDES**

| Nom INCI | Quantité |
|---|---|
| WATER | QSP 100 |
| GLYCERIN | 5 |
| CARBOMER | 0.2 |
| TETRASODIUM EDTA | 0.1 |
| CAMELLIA SINENSIS LEAF OIL | 2 |
| HYDROGENATED POLYISOBUTENE | 8 |
| GLYCERYL STEARATE SE | 2 |
| DIMETHICONE | 1 |
| GLYCERYL STEARATE AND PEG-100 STEARATE | 1.5 |
| TRIETHYLHEXANOIN | 5 |
| STEARIC ACID | 2 |
| CETYL ALCOHOL | 1 |
| SILICA | 1 |
| DICAPRYLYL MALEATE | 6 |
| GLYCERYL STEARATE | 1 |
| DIMETHICONE | 3.5 |
| WATER | 2.3 |
| TRIETHANOLAMINE | 0.5 |
| PHENOXYETHANOL, METHYLPARABEN, | |
| PROPYLPARABEN, BUTYLPARABEN, ETHYLPARABEN | 0.7 |
| FRAGRANCE | 0.3 |
| TOCOPHERYL ACETATE | 0.5 |
| RETINOL | 0.1 |
| SODIUM HYALURONATE | 0.03 |
| CENTELLA ASIATICA EXTRACT | 1 |
| HYDROLYZED SOY PROTEIN | 0.4 |
| FRACTIONS ACIDES GANODERIQUES | 1 |

**SERUM ANTI-TACHES**

| Nom INCI | Quantité |
|---|---|
| WATER | QSP 100 |
| TRISODIUM EDTA | 0.1 |
| HYDROXYETHYLCELLULOSE | 0.1 |
| XANTHAN GUM | 0.3 |
| PHENOXYETHANOL, METHYLPARABEN, PROPYLPARABEN, BUTYLPARABEN, ETHYLPARABEN | 0.65 |
| BUTYLENE GLYCOL | 5 |
| POLYSORBATE 20 | 1 |
| FRAGRANCE | 0.05 |
| TOCOPHEROL ACETATE | 0.1 |
| SODIUM CITRATE | 0.65 |
| MAGNESIUM ASCORBYL PHOSPHATE | 1 |
| FRACTIONS ACIDES GANODERIQUES | 3 |

**FOND DE TEINT**

| Nom INCI | Quantité |
|---|---|
| WATER | QSP 100 |
| MAGNESIUM ALUMINIUM SILICATE | 0.5 |
| CELLULOSE GUM | 0.35 |
| GLYCERIN | 3 |
| PVP | 5 |
| DIPROPYLENE GLYCOL | 0.05 |
| PROPYLENE GLYCOL | 2 |
| PHENOXYETHANOL, METHYLPARABEN, PROPYLPARABEN, BUTYLPARABEN, ETHYLPARABEN | 1 |
| XANTHAN GUM | 0.2 |
| TRIETHANOLAMINE | 0.7 |
| ISOPROPYL PALMITATE | 4 |
| MINERAL OIL | 2 |
| HEXYLDECANOL | 3 |
| GLYCERYLSTEARATE | 2 |
| STEARIC ACID | 2.4 |
| OLEIC ACID | 0.5 |
| POLYSORBATE 60 | 0.7 |
| TOCOPHEROL | 0.5 |
| TITANIUM DIOXIDE | 7 |
| IRON OXIDE | 4 |
| NYLON-2 | 6 |
| SODIUM HYALURONATE | 0.01 |
| FRAGRANCE | 0.5 |
| PROPYLENE GLYCOL DICAPRYLATE/DICAPRATE | 7.2 |
| FRACTIONS ACIDES GANODERIQUES | 1 |

**EMULSION BLANCHISSANTE PROTECTRICE UVA/UVB**

| Nom INCI | Quantité |
|---|---|
| OCTYLMETHOXYCINNAMATE | 4 |
| CETYL PEG/PPG-10/1 DIMETHICONE | 3 |
| Bis-PEG/PPG-14/14 DIMETHICONE | 3 |
| DIMETHICONE | 6 |
| CYCLOMETHICONE | 4 |
| POLYDECENE | 4 |
| METHYLPARABEN, PROPYLPARABEN, BUTYLPARABEN, ETHYLPARABEN | 0.65 |
| TOCOPHEROL | 0.5 |
| FRAGRANCE | 0.8 |
| PPG-3 MYRISTYL ETHER | 0.5 |
| TITANIUM DIOXYDE | 8 |
| PHENOXYETHANOL | 0.35 |
| SODIUM CITRATE | 0.65 |
| MAGNESIUM ASCORBYL PHOSPHATE | 3 |
| WATER | QSP 100 |
| XANTHAN GUM | 0.4 |
| BUTYLENE GLYCOL | 2 |
| FRACTIONS ACIDES GANODERIQUES | 1 |

**GEL AMINCISSANT**

| Nom INCI | Quantité |
|---|---|
| WATER | QSP 100 |
| TETRASODIUM EDTA | 0.2 |
| CARBOMER | 0.5 |
| GLYCERINE | 3.0 |
| POLYGLYCERYLMETHACRYLATE AND PROPYLENE GLYCOL | 5.0 |
| DIPROPYLENE GLYCOL | 3.0 |
| BUTYLENE GLYCOL | 5.0 |
| PHENOXYETHANOL, METHYLPARABEN, PROPYLPARABEN, BUTYLPARABEN, ETHYLPARABEN | 0.65 |
| TRIETHNOLAMINE | 0.5 |
| CAFEINE | 2 |
| RUSCUS ACULEATUS EXTRACT | 1 |
| FRACTIONS ACIDES GANODERIQUES | 1 |

**CREME HYDRATANTE**

| Nom INCI | Quantité |
|---|---|
| WATER | QSP 100 g |
| CARBOMER | 0.35 |
| TETRASODIUM EDTA | 0.1 |
| POLYSORBATE 60 | 3 |
| SORBITAN STEARATE | 2.6 |
| ISOPROPYL PALMITATE | 2.5 |
| CETYL PALMITATE | 4 |
| ETHYLHEXYL PALMITATE | 5.1 |
| SQUALANE | 1 |
| CETYL ALCOHOL | 2.5 |
| CYCLOMETHICONE | 1 |
| DIMETHICONE | 0.5 |
| TRIETHANOLAMINE | 0.53 |
| BUTYLENE GLYCOL | 5 |
| FRAGRANCE | 0.3 |
| PHENOXYETHANOL, METHYLPARABEN, ETHYLPARABEN, PROPYLPARABEN, BUTYLPARABEN, | 0.65 |
| TOCOPHERYL ACETATE | 0.5 |
| SODIUM HYALURONATE | 0.03 |
| SWEET ALMOND PROTEIN AND GLYCERIN | 2 |
| POLYGLYCERYLMETHACRYLATE | 5 |
| FRACTIONS ACIDES GANODERIQUES | 1 |

Dans chacune de ces formulations, la fraction d'acide ganodérique incorporée (qui se présente sous la forme de poudre par exemple) est de l'ordre de 1%, étant entendu que cette fraction peut varier dans des proportions bien connues de l'homme du métier, et par exemple entre 0,5 et 5%, sans pour autant sortir du cadre de l'invention.

### POSSIBILITE D'APPLICATION INDUSTRIELLE

L'invention trouve son application industrielle dans la fabrication de compositions pharmaceutiques, cosmétiques ou alimentaires.

## Revendications

1. - Utilisation de composés ayant une structure triterpénoïde avec deux fonctions méthyles en position 4 et au moins une fonction hydroxyle en positions 3 et 7, lesdits composés comprenant des acides ganodériques choisis parmi les espèces B, C₂, G, seuls ou en combinaison, en tant qu'agent cosmétique ayant des effets anti-vieillissement.

2. - Utilisation selon la revendication 1 **caractérisée en ce que** les effets anti-vieillissement sont des effets protecteurs sur l'apoptose cortico-induite.

3. - Utilisation selon la revendication 1 **caractérisée en ce que** lesdits composés à structure triterpénoïde ont des effets amincissants localisés.

4. - Utilisation selon l'une des revendications précédentes **caractérisée en ce que** les acides ganodériques proviennent d'un extrait de champignon de la famille des *ganodermataceaes* (polyporacés).

5. - Utilisation selon la revendication 4 **caractérisée en ce que** l'extrait de champignon provient du *ganoderma lucidum.*

6. - Utilisation selon la revendication 5 **caractérisée en ce que** l'extrait d'acides ganodériques comprend un mélange d'acides ganodériques B, C₂ et G.

7. - Utilisation de composés ayant une structure triterpénoïde comportant au moins une fonction hydroxyle en positions 3 et 7, lesdits composés comprenant un mélange d'acides ganodériques B et C₂, ou un mélange d'acides ganodériques B et G, ou un mélange d'acides ganodériques C₂ et G, pour la préparation d'un médicament, en vue du traitement ou de la prévention d'un désordre cutané.

8. - Utilisation selon la revendication 7 **caractérisée en ce qu'**elle consiste à traiter ou prévenir l'apoptose cortico-induite.

9. - Utilisation selon la revendication 7 **caractérisée en ce qu'**elle consiste à traiter ou prévenir des désordres résultant d'inflammations cutanées ou du métabolisme adipocytaire.

10. - Composition comprenant des acides ganodériques **caractérisée en ce qu'**elle comprend un mélange d'au moins une fraction d'acide ganodérique B et d'une fraction d'acide ganodérique C₂, ou un mélange d'une fraction d'acide ganodérique B et d'une fraction d'acide ganodérique G, ou un mélange d'une fraction d'acide ganodérique C₂ et d'une fraction d'acide ganodérique G, lesdites fractions étant supérieures à 15% au sein de la fraction totale des acides ganodériques.

11. - Composition selon la revendication 10 **caractérisée en ce qu'**elle comprend un mélange d'acides ganodériques B, C₂ et G, dont la proportion est supérieure à 15% au sein de la fraction totale d'acides ganodériques.

12. - Composition selon l'une des revendications 10 à 11 **caractérisée en ce qu'**elle est formée par une composition alimentaire comprenant les excipients appropriés pour une utilisation alimentaire.

13. - Composition selon l'une des revendications 10 à 11 **caractérisée en ce qu'**elle est formée par une composition cosmétique comprenant les excipients appropriés pour une utilisation cosmétique.

14. - Composition selon l'une des revendications 10 à 11 **caractérisée en ce qu'**elle est formée par une composition pharmaceutique comprenant les excipients appropriés pour une utilisation pharmaceutique.

## Patentansprüche

1. - Verwendung von Zusammensetzungen mit einer triterpenoiden Struktur mit zwei Methylfunktionen in 4-Stellung und mindestens einer Hydroxylfunktion in der 3- und 7-Stellung, wobei die Zusammensetzungen Ganodericsäuren aufweisen, die aus den Artengruppen B, C₂, G einzeln oder in Kombination als kosmetisches Mittel mit Anti-Alterungswirkungen ausgewählt sind.

2. - Verwendung nach Anspruch 1, **dadurch gekennzeichnet, dass** die Anti-Alterungswirkungen Schutzwirkungen gegen kortikoinduzierte Apoptose haben.

3. - Verwendung nach Anspruch 1, **dadurch gekennzeichnet, dass** die Zusammensetzungen mit triterpenoider Struktur lokale schlankmachende Wirkungen aufweisen.

4. - Verwendung nach einem der vorhergehenden Ansprüche, **dadurch gekennzeichnet, dass** die Ganodericsäuren aus einem Extrakt aus dem Pilz der Familie der *Ganodermataceae* (Polyporaceae) stammen.

5. - Verwendung nach Anspruch 4, **dadurch gekennzeichnet, dass** der Pilzextrakt aus dem *Ganoderma lucidum* stammt.

6. - Verwendung nach Anspruch 5, **dadurch gekennzeichnet, dass** der Extrakt aus Ganodericsäuren eine Mischung aus den Ganodericsäuren B, C₂ und G aufweist.

7. - Verwendung von Zusammensetzungen mit einer triterpenoiden Struktur mit mindestens einer Hydroxylfunktion in der 3- und 7-Stellung, wobei die Zusammensetzungen eine Mischung von Ganodericsäuren B und C₂ oder eine Mischung von Ganodericsäuren B und G oder eine Mischung von Ganodericsäuren C₂ und G für die Zubereitung eines Medikaments für die Behandlung oder Vorbeugung einer Hauterkrankung aufweisen.

8. - Verwendung nach Anspruch 7, **dadurch gekennzeichnet, dass** sie darin besteht, die kortikoinduzierte Apoptose zu behandeln oder ihr vorzubeugen.

9. - Verwendung nach Anspruch 7, **dadurch gekennzeichnet, dass** sie darin besteht, Erkrankungen aufgrund von Hautentzündungen oder adipozytärem Stoffwechsel zu behandeln oder ihnen vorzubeugen.

10. - Zusammensetzung, die Ganodericsäuren aufweist, **dadurch gekennzeichnet, dass** sie eine Mischung aus mindestens einer Fraktion der Ganodericsäure B und einer Fraktion der Ganodericsäure C₂, oder eine Mischung aus einer Fraktion der Ganodericsäure B und einer Fraktion der Ganodericsäure G oder eine Mischung aus einer Fraktion der Ganodericsäure C₂ und einer Fraktion der Ganodericsäure G aufweist, wobei die Fraktionen höher als 15% innerhalb der Gesamtfraktion der Ganodericsäuren sind.

11. - Zusammensetzung nach Anspruch 10, **dadurch gekennzeichnet, dass** sie eine Mischung aus Ganodericsäuren B, C₂ und G aufweist, deren Anteil höher als 15 % innerhalb der Gesamtfraktion der Ganodericsäuren ist.

12. - Zusammensetzung nach einem der Ansprüche 10 bis 11, **dadurch gekennzeichnet, dass** sie aus einer Nahrungsmittelzusammensetzung gebildet ist, die die für eine Verwendung als Nahrungsmittel geeigneten Hilfsstoffe aufweist.

13. - Zusammensetzung nach einem der Ansprüche 10 bis 11, **dadurch gekennzeichnet, dass** sie aus einer kosmetischen Zusammensetzung gebildet ist, die die für eine kosmetische Verwendung geeigneten Hilfsstoffe aufweist.

14. - Zusammensetzung nach einem der Ansprüche 10 bis 11, **dadurch gekennzeichnet, dass** sie aus einer pharmazeutischen Zusammensetzung gebildet ist, die die für eine pharmazeutische Verwendung geeigneten Hilfsstoffe aufweist.

## Claims

1. - Use of compounds having a triterpenoid structure with two methyl functions in position 4 and at least one hydroxyl function in positions 3 and 7, said compounds comprising ganoderic acids chosen from the species B, C₂, G, alone or in combination, as a cosmetic agent having anti-aging effects.

2. - Use according to claim 1, **characterised in that** the anti-aging effects are protective effects on cortico-induced apoptosis.

3. - Use according to claim 1, **characterised in that** said compounds with a triterpenoid structure have localised thinning effects.

4. - Use according to one of the preceding claims, **characterised in that** the ganoderic acids come from an extract of fungus in the *Ganodermataceae* (*Polyporaceae*) family.

5. - Use according to claim 4, **characterised in that** the extract of fungus comes from *Ganoderma lucidum.*

6. - Use according to claim 5, **characterised in that** the extract of ganoderic acids comprises a mixture of ganoderic acids B, C₂ and G.

7. - Use of compounds having a triterpenoid structure comprising at least one hydroxyl function in positions 3 and 7, said compounds comprising a mixture of ganoderic acids B and C₂, or a mixture of ganoderic acids B and G, or a mixture of ganoderic acids C₂ and G, for preparing a medication, with a view to the treatment or prevention of a skin problem.

8. - Use according to claim 7, **characterised in that** it consists of treating or preventing cortico-induced apoptosis.

9. - Use according to claim 7, **characterised in that** it consists of treating or preventing problems resulting from skin inflammations or adipose metabolism.

10. - Composition comprising ganoderic acids, **characterised in that** it comprises a mixture of at least one fraction of ganoderic acid B and one fraction of ganoderic acid C₂, or a mixture of a fraction of ganoderic acid B and a fraction of ganoderic acid G, or a mixture of a fraction of ganoderic acid C₂ and a fraction of ganoderic acid G, said fractions being greater than 15% in the total fraction of ganoderic acids.

11. - Composition according to claim 10, **characterised in that** it comprises a mixture of ganoderic acids B, C₂ and G, the proportion of which is greater than 15% in the total fraction of ganoderic acids.

12. - Composition according one of claims 10 to 11, **characterised in that** it is formed by a food composition comprising the appropriate excipients for food use.

13. - Composition according to one of claims 10 to 11, **characterised in that** it is formed by a cosmetic composition comprising the appropriate excipients for cosmetic use.

14. - Composition according to one of claims 10 to 11, **characterised in that** it is formed by a pharmaceutical composition comprising the appropriate excipients for pharmaceutical use.
